(19) Europäisches Patentamt
European Patent Office
Office européen des brevets

(11) Numéro de publication: **0 034 556**
**B1**

# (12) FASCICULE DE BREVET EUROPEEN

(45) Date de publication du fascicule du brevet: **01.02.84**

(51) Int. Cl.³: **C 07 C 103/58,** A 01 N 37/30

(21) Numéro de dépôt: **81420020.0**

(22) Date de dépôt: **17.02.81**

(54) **Compositions régulatrices de la croissance des plantes contenant comme matière active des dérivés de l'acide méthylène-2 succinamique et nouveaux dérivés de cet acide utilisables dans ces compositions.**

(30) Priorité: **18.02.80 FR 8003876**

(43) Date de publication de la demande:
**26.08.81 Bulletin 81/34**

(45) Mention de la délivrance du brevet:
**01.02.84 Bulletin 84/5**

(84) Etats contractants désignés:
**AT BE CH DE FR GB IT LI LU NL**

(56) Documents cités:
**BE - A - 732 138**
**FR - A - 1 310 442**
**FR - A - 2 330 317**
**US - A - 3 510 509**

**CHEMICAL ABSTRACTS, vol. 93, no. 63475z, no. 7, 18 août 1980 Columbus, Ohio, US E. KARANOV et al.: "Herbicidal activity and chemical structure of some substituted itaconic acid anilides"**
**J. ORG. CHEM. vol. 28, août 1963, Washington, US A. ZIKHA et al.: "Syntheses of Amide Derivatives of DL-beta-Carboxy-gamma-aminobutyric Acid", pages 2007-9**

(73) Titulaire: **RHONE-POULENC AGROCHIMIE, 14-20, rue Pierre Baizet, F-69009 Lyon (FR)**

(72) Inventeur: **Disdier, André, 1 rue du Tonkin, F-69100 Villeurbanne (FR)**
Inventeur: **Borrod, Guy, 38 bis rue des Granges, F-69005 Lyon (FR)**
Inventeur: **Trinh, Stéphane, 25 Boulevard de la République, F-69410 Champagne au Mont d'Or (FR)**

(74) Mandataire: **Chaumette, Michel et al, RHONE-POULENC AGROCHIMIE BP 9163-09, F-69263 Lyon Cedex 1 (FR)**

**EP 0 034 556 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Compositions régulatrices de la croissance des plantes contenant comme matière active des dérivés de l'acide méthylène-2 succinamique et nouveaux dérivés de cet acide utilisables dans ces compositions

La présente invention concerne des compositions régulatrices de la croissance des plantes contenant comme matière active des dérivés de l'acide méthylène-2 succinamique ainsi que les traitements régulateurs de la croissance des plantes effectués au moyen de ces dérivés. Elle concerne, de plus, de nouveaux dérivés de l'acide méthylène-2 succinamique, utilisables comme matière active dans les compositions et les traitements, selon l'invention, ainsi que la préparation de ces nouveaux dérivés.

Dans le texte de la présente demande de brevet, les expressions »régulatrices de la croissance« et »régulateurs de croissance« sont prises dans leur acception courante en langue française, qui correspond à »substances de croissance« de la littérature anglo-saxonne, le mot »croissance« se rapportant à la production de matière vivante et non simplement à la modification de la taille des plantes. Par »régulateurs de croissance«, on entendra donc dans la suite des produits capables de modifier la physiologie des plantes de diverses manières.

Dans le domaine de l'agriculture, l'amélioration des rendements des cultures constitue un objectif permanent des agriculteurs notamment lorsqu'il s'agit de cultures importantes sur le plan économique comme, par exemple, celle du soja. Un moyen de tendre vers ce but est de lutter contre les parasites et prédateurs de ces cultures en y détruisant les mauvaises herbes et les insectes nuisibles au moyen de désherbants sélectifs et d'insecticides appropriés, et également de combattre les maladies fongiques de ces cultures au moyen de produits antifongiques appropriés.

Indépendamment de ces moyens couramment utilisés, il est très souhaitable de pouvoir agir sur les cultures elles-mêmes pour en stimuler ou orienter le développement physiologique de façon à améliorer les rendements.

Un but de la présente invention est de répondre à ce besoin en mettant à la disposition de l'agriculture des produits et compositions permettant d'augmenter le rendement des cultures grâce à leur action sur le mode de croissance des plantes, notamment en ce qui concerne le soja.

Il a maintenant été trouvé que ce but pouvait être atteint grâce à de nouvelles compositions régulatrices de la croissance des plantes, caractérisées en ce qu'elles contiennent comme matière active:

au moins un composé de formule I:

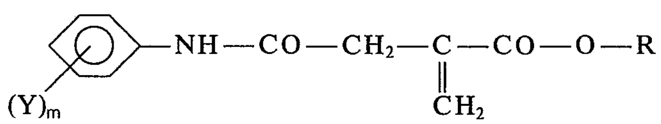

(I)

dans laquelle:

- R représente un atome d'hydrogène ou un radical alcoyle contenant de 1 à 16 atomes de carbone, ou alcényle contenant de 3 à 15 atomes de carbone, ou alcynyle contenant de 3 à 5 atomes de carbone,
- Y représente un atome d'halogène ou un radical alcoyle comportant de 1 à 4 atomes de carbone,
- m est un nombre entier de 0 à 5 ($0 \leq m \leq 5$) étant entendu que, lorsque m est supérieur à 1, les substituants Y peuvent être identiques ou différents,

ou, lorsque R représente un atome d'hydrogène, un sel acceptable en agriculture de ce composé.

Dans ce qui suit, on entendra par »sels acceptables en agriculture« des sels qui comportent des cations connus et utilisables dans la pratique pour la préparation des sels, destinés à être utilisés en agriculture ou horticulture, des acides carboxyliques à propriétés pesticides.

Les sels du composé de formule I utilisables selon l'invention peuvent être représentés d'une façon générale par la formule:

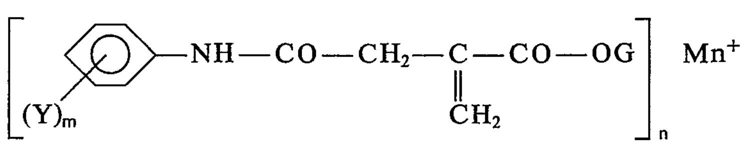

(II)

dans laquelle:

- M représente un cation minéral ou organique de valence n, et
- Y et m ont même signification que dans la formule I.

Comme sels du composé de formule I utilisables dans les compositions selon l'invention, on peut mentionner plus particulièrement les sels de métaux alcalins ou alcalino-terreux ainsi que les sels d'amines. Ces derniers répondent à la formule (II) dans laquelle n est égal à 1 et $M^{n+}$ represente le cation de formule:

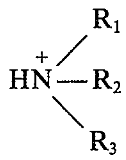

dans laquelle $R_1$, $R_2$ et $R_3$, identiques ou différents représentent chacun un atome d'hydrogène ou un radical alcoyle contenant de 1 à 18 atomes de carbone et éventuellement substitué, par exemple par un hydroxy.

Les sels de métaux alcalins ou alcalino-terreux sont généralement obtenus par simple salification de l'acide répondant à la formule I (lorsque R représente l'atome d'hydrogène) au moyen d'une base appropriée, ou par réaction de double décomposition entre un sel dudit acide et un autre sel comportant le cation souhaité.

Les sels d'amine sont généralement obtenus par action de l'acide répondant à la formule I (lorsque R représente l'atome d'hydrogène) avec une amine primaire, secondaire ou tertiaire appropriée, par exemple avec l'éthanolamine. L'utilisation d'amines à longue chaîne du type amines grasses permet d'obtenir des sels hydro et/ou liposolubles.

L'invention concerne plus particulièrement les compositions régulatrices de la croissance des plantes contenant comme matière active au moins un composé répondant à la formule I citée plus haut dans laquelle m est égal à o, 1, 2 ou 3, Y représente un atome d'halogène, de préférence l'atome de chlore, et R a même signification que dans cette formule, ou au moins un sel acceptable en agriculture de ce composé.

Parmi ces composés, des sous-familles préférées comprennent les composés répondant aux formules III et III bis ci-après:

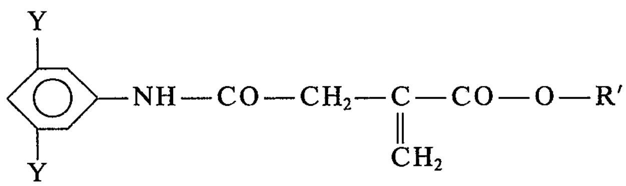

(III)

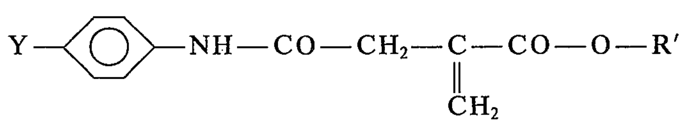

(III bis)

dans lesquelles Y représente un atome d'halogène, de préférence le chlore, et R' représente l'atome d'hydrogène ou un radical alcoyle contenant de 1 à 8 atomes de carbone ou alcényle contenant de 3 à 5 atomes de carbone, ainsi que les sels acceptables en agriculture de ces composés (tout particulièrement leurs sels d'amines).

Parmi les composés mentionnés précédemment, d'excellents résultats ont été obtenus en utilisant comme matière active dans les compositions selon l'invention les composés répondant à la formule III dans laquelle Y représente un atome de chlore et R' représente l'atome d'hydrogène ou un radical alcényle comportant de 3 à 5 atomes de carbone, comme par exemple le radical allyle, ainsi que les sels acceptables en agriculture de ces composés.

Certains des composés selon la formule I ont déjà été décrits dans la littérature ainsi:

- la référence J. Org. Chem. — Vol 28 — août 1963, p. 2007-9 décrit l'acide N-phényl-méthylène-2-succinamique,
- le brevet français No 1 310 442 revendique en tant qu'herbicides des N-arylitaconimides, distincts des composés selon l'invention, mais préparés à partir d'acides N-arylitaconamiques eux-mêmes obtenus par action d'une amine aromatique sur l'anhydride itaconique,
- le brevet belge No 732 138 et son équivalent anglais No 1 209 005 revendiquent en tant qu'agent antimicrobien et antifongique le N-(dichloro-3,5 phényl)-itaconimide, distinct des composés selon l'invention, mais préparé à partir de l'acide N-(dichloro-3,5 phényl) itaconique.

Ces documents décrivent donc certains des acides répondant à la formule I (lorsque R représente l'hydrogène). Toutefois, aucun de ces documents ne mentionne ni ne suggère la possibilité d'une activité régulatrice de la croissance des plantes pour ces acides, ni même une quelconque utilisation

de ces acides comme matière active dans des compositions à usage agricole.

En plus des compositions mentionnées plus haut, l'invention concerne, en tant que produits nouveaux, les composés répondant à la formule I et leurs sels acceptables en agriculture, à l'exclusion toutefois des acides répondant à la formule I (pour lesquels R représente l'atome d'hydrogène). Elle concerne tout particulièrement ceux pour lesquels m est égal à 0, 1, 2 ou 3, Y représente un atome d'halogène ainsi que les sels acceptables en agriculture de ces composés.

De préférence, l'invention concerne en tant que produits nouveaux les composés répondant aux formules III et III bis dans lesquelles Y représente un atome d'halogène, de préférence le chlore et R' représente un radical alcoyle contenant de 1 à uatomes de carbone ou alcényle contenant de 3 à 5 atomes de carbone, ainsi que les sels acceptables en agriculture de ces composés. Elle concerne plus particulièrement les composés répondant à la formule III dans laquelle R' représente un radical alcényle comportant de 3 à 5 atomes de carbone tel que le radical allyle.

Les composés selon la formule I, pour lesquels R représente un atome d'hydrogène peuvent être obtenus par action d'une aniline sur l'anhydride itaconique, selon le schéma réactionnel en soi connu:

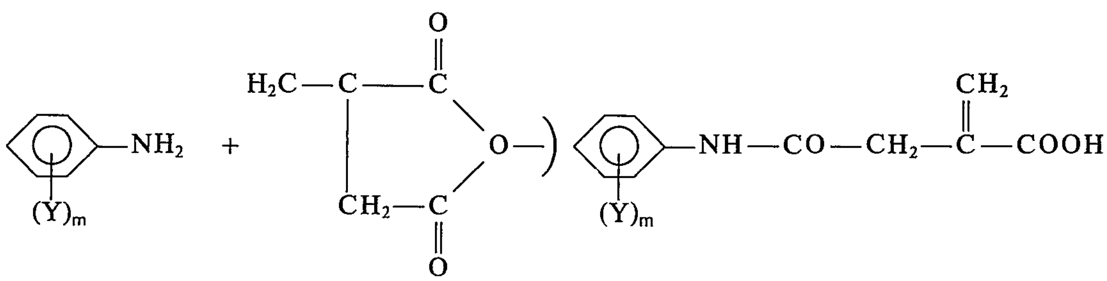 (IV)

dans lequel Y et m ont la même signification que dans la formule I.

La réaction s'effectue en milieu anhydre, avantageusement dans un solvant inerte (c'est-à-dire inerte chimiquement vis-à-vis des réactifs et produits de la réaction, dans les conditions opératoires), à une température comprise entre 20 et 150° C environ.

Comme solvant approprié, on peut citer des solvants organiques, polaires ou non polaires, tels que des hydrocarbures aromatiques (tels que le benzène, le toluène, les xylènes, le chlorobenzène), des hydrocarbures saturés, cycliques ou aliphatiques (tels que l'hexane, l'heptane, le cyclohexane, le méthylcyclohexane), des hydrocarbures aliphatiques chlorés (tels que le dichloroéthane, le dichloroéthylène, le tétrachlorure de carbone, le perchloréthylène), des éthers (tels que le dioxanne, le tétrahydrofurane, l'éther diéthylique, l'éther diisopropylique), des cétones (telles que l'acétone, la méthyl-éthylcétone, la méthyl-isobutylcétone), ou des nitriles (tels que l'acétonitrile).

En fin de réaction, l'acide IV formé peut être isolé du milieu réactionnel par tout moyen en soi connu tel que, par exemple, par distillation du solvant ou par cristallisation du produit dans le milieu réactionnel, puis, si nécessaire, cet acide est purifié par des méthodes usuelles telles que recristallisation dans un solvant approprié, chromatographie en phase liquide.

Les composés selon la formule I pour lesquels R représente un radical alcoyle, alcényle ou alcynyle peuvent être obtenus à partir des acides selon la formule (IV), par estérification au moyen d'un alcool approprié, selon le schéma réactionnel:

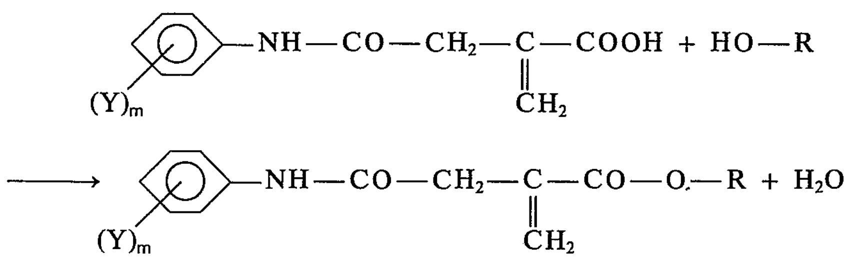

(IV)

(V)

dans lequel Y et m ont la même signification que dans la formule I et R représente un radical alcoyle contenant de 1 à 16 atomes de carbone alcényle contenant de 3 à 15 atomes de carbone, ou alcynyle contenant de 3 à 5 atomes de carbone.

La réaction s'effectue en milieu anhydre, à une température comprise entre 50 et 160° C, en présence d'un catalyseur du type acide de Lewis tel que, par exemple l'acide chlorhydrique, l'acide sulfurique, les acides aryl ou alkylsulfoniques (et notamment l'acide méthane-sulfonique et l'acide p-toluène-sulfonique).

Avantageusement la réaction est effectuée dans un solvant inerte tels que, par exemple ceux utilisés dans la préparation de l'acide de formule IV, et dont la liste a été donnée plus haut. Cette réaction peut également être effectuée dans un excès d'alcool de formule R – OH, qui sert alors à la fois de réactif et de solvant, avec ou sans utilisation conjointe d'un autre solvant.

Pour faciliter la réaction en déplaçant l'équilibre vers la droite, il est avantageux d'éliminer l'eau formée, par distillation azéotropique, à partir du mélange ternaire solvant/alcool/eau ou du mélange binaire alcool/eau.

En fin de réaction, les produits formés sont isolés par tout moyen en soi connu tel que par exemple, par filtration, ou par distillation du solvant ou par cristallisation du produit dans le milieu réationnel, puis si nécessaire, ils sont purifiés selon les méthodes usuelles.

Les composés selon la formule I pour lesquels R représente un radical alcoyle, alcényle ou alcynyle peuvent également être préparés selon un deuxième procédé qui consiste à faire réagir un sel de l'acide de formule IV (de préférence un sel de sodium ou d'ammonium) avec un halogénure de formule XR dans laquelle R a même signification que précédemment et X représente un atome d'halogène (de préférence un atome de brome ou de chlore). La réaction s'effectue en milieu solvant organique ou hydroorganique, à une température généralement comprise entre 20 et 100°C. Comme solvants utilisables, on peut mentionner ceux cités plus haut dans le cas de la préparation de l'acide de formule IV. En fin de réaction, les produits formés sont isolés par tout moyen connu tels que ceux mentionnés précédemment, puis, si nécessaire, purifiés par les méthodes usuelles.

Les sels de métaux alcalin ou alcalino-terreux ou d'ammonium de l'acide de formule IV peuvent être obtenus par action sur cet acide d'une base telle que la soude ou l'ammoniaque, généralement en milieu aqueux ou hydroorganique dès la température ordinaire. Les sels d'amine de l'acide de formule IV peuvent être obtenus par action dudit acide sur une amine appropriée, à chaud en milieu solvant organique ou hydroorganique.

Les exemples décrits ci-après illustrent l'invention sans toutefois la limiter. Les composés décrits dans les exemples 1 à 14 ont été identifiés par spectrométrie de résonnance magnétique nucléaire (RMN), les spectres ayant été réalisés à 60 mégahertz dans $CCl_4$ ou $CDCl_3$, avec l'hexaméthyldisiloxane comme référence interne.

Dans le cas des composés 7, 10 et 14, le dosage de la fonction carboxylique (dosage potentiographique en présence de soude) et de la fonction ammonium (dosage par l'acide perchlorique en présence d'acétate de mercure) ont donné respectivement les résultats ci-après:

- composé No 7: 92,0% – 95,3%,
- composé No 10: 94,5% – 91,3%,
- composé No 14: 98,8% – 92,3%.

Exemple 1

Préparation de l'acide N-(dichloro-3,5 phényl)-méthylène-2 succinamique (composé No 1)

Dans 300 ml de toluène, on dissout 33,6 g d'anhydride itaconique (0,3 mole), à 60°C environ et on coule lentement dans cette solution une solution de 48,6 g (0,3 mole) de dichloro-3,5 aniline dans 200 ml de toluène. On observe l'apparition d'un précipité. Le mélange réactionnel est maintenu sous agitation, à température ambiante (15 à 25°C) pendant deux heures.

Le produit formé est séparé par filtration puis séché. On obtient ainsi 66,6 g d'acide N-(dichloro-3,5 phényl)-méthylène-2 succinamique fondant à 185°C, soit un rendement de 81%.

Composition élémentaire

| | calculée | trouvée |
|---|---|---|
| C % | 48,17 | 48,00 |
| H % | 3,28 | 3,11 |
| N % | 5,10 | 5,22 |
| Cl % | 25,91 | 25,81 |

L'anhydride itaconique et l'aniline de départ sont des produits commerciaux.

Exemple 2

Préparation du N-(dichloro-3,5 phényl)-méthylène-2 succinamate d'allyle (composé No 2)

On mélange, à température ambiante (15 à 25°C), 27,4 g (0,1 mole) d'acide N-(dichloro-3,5 phényl)-méthylène-2 succinamique, 200 ml d'alcool allylique et 1 ml d'acide sulfurique concentré,

jusqu'à obtention d'une solution. Cette solution est ensuite chauffée à reflux, pendant 2 heures avec distillation du mélange alcool allylique/eau. Tout au long de la distillation on ajoute de l'alcool allylique à la solution de façon à remplacer celui qui est éliminé par distillation. Après refroidissement à température ambiante, l'excès d'alcool allylique est évaporé sous vide partiel de la trompe à eau. Le résidu est dissous dans 200 ml de chlorure de méthylène, lavé par 200 ml d'eau, puis par 100 ml d'une solution de bicarbonate de sodium à 5% et enfin par 200 ml d'eau. Après séchage sur sulfate de sodium anhydre et élimination du chlorure de méthylène par évaporation, le résidu est recristallisé dans 150 ml d'éther diéthylique et on obtient ainsi 14,8 g de N-(dichloro-3,5 phényl)-méthylène-2 succinamate d'allyle, fondant à 99° C, soit un rendement de 47,1%.

Composition élémentaire

| | calculée | trouvée |
|---|---|---|
| C % | 53,50 | 53,51 |
| H % | 4,14 | 4,03 |
| N % | 4,45 | 4,48 |
| Cl % | 22,61 | 22,19 |

La préparation de l'acide N-(dichloro-3,5 phényl)-méthylène-2 succinamique est décrite dans l'exemple 1.

Exemples No 3 et 4

En opérant comme à l'exemple 2, à partir de l'acide N-(dichloro-3,5 phényl)-méthylène-2 succinamique et d'un alcool approprié, les composés suivants ont été préparés:

- N-(dichloro-3,5 phényl)-méthylène-2 succinamate de méthyle (composé No 3), fondant à 113–114° C.
- N-(dichloro-3,5 phényl)-méthylène-2 succinamate d'isopropyle (composé No 4), fondant a 93° C.

Exemple No 5

Préparation du N-(dichloro-3,5 phényl)-méthylène-2 succinamate de n-butyle (composé No 5)

On dissout 11 g d'acide N-(dichloro-3,5 phényl)-méthylène-2 succinamique dans une solution de 1,6 g de soude dans 50 ml d'eau et il se forme ainsi le sel de sodium de cet acide.

A cette solutiuon, on ajoute 5,5 g de bromure de n-butyle en solution dans 100 ml d'éthanol. Le mélange réactionnel obtenu est chauffé pendant 16 heures, à reflux du mélange eau/éthanol. On extrait ensuite l'huile formée avec 200 ml de chlorure de méthylène. Après séchage sur sulfate de sodium anhydre et évaporation du solvant, on obtient une huile qui cristallise à température ambiante. Le produit brut obtenu est recristallisé dans 100 ml de cyclohexane. On obtient ainsi 2,8 g de N-(dichloro-3,5 phényl)-méthylène-2 succinamate de n-butyle fondant à 69,5° C. Rendement: 21,2%.

Exemple No 6

En opérant comme à l'exemple 5 à partir du même acide et de bromure de n-hexyle, on obtient du N-(dichloro-3,5 phényl)-méthylène-2 succinamate de n-héxyle (composé No 6) fondant à 60°C. Rendement: 13,3%.

Exemple No 7

Préparation du N-(dichloro-3,5 phényl)-méthylène-2 succinamate d'éthanolamine (composé No 7), de formule

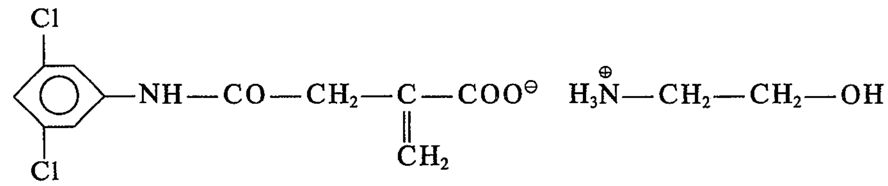

On mélange 13,7 g d'acide N-(dichloro-3,5 phényl)-méthylène-2 succinamique, 3,05 d'éthanolamine et 50 ml d'éthanol et le mélange obtenu est chauffé pendant 30 minutes à reflux de l'éthanol. Après évaporation à sec, on obtient un produit brut très visqueux que l'on sèche à 50°C sous pression de $5 \cdot 10^{-2}$ mm.Hg.

On obtient ainsi 16,5 g du produit cherché, sous forme d'un liquide fisqueux soluble dans l'eau. Rendement: 98,5%.

Exemples No 8, 9 et 10

En opérant respectivement selon les méthodes décrites dans les exemples No 1, 2 et 7, à partir des matières premières appropriées, les composés ci-après ont été préparés:

- acide N-(chloro-4 phényl)-méthylène-2 succinamique (composé No 8) fondant à 182–183°C. Rendement: 95%,
- N-(chloro-4 phényl)-méthylène-2 succinamate d'allyle (composé No 9) fondant à 82,3°C. Rendement: 17%,
- N-(chloro-4 phènyl)-méthylène-2 succinamate d'éthanolamine (composé No 10), obtenu sous forme d'un liquide visqueux, soluble dans l'eau. Rendement: 99,2%.

Exemples No 11 et 12

En opérant selon les méthodes décrites respectivement dans les exemples No 1 et 2, à partir des matières premiérés appropriées, les composés ci-après ont été préparés:

- acide N-(dichloro-3,4 phényl)-méthylène-2 succinamique (composé No 11) fondant à 158°C,
- N-(dichloro-3,4 phényl)-méthylène-2 succinamate de méthyle (composé No 12) fondant à 111,7°C. Rendement: 42,4%.

Exemples 13 et 14

En opérant selon les méthodes décrites respectivement dans les exemples No 1 et 7, à partir des matières premières appropriées, les composés ci-après ont été préparés:

- acide N-(méthyl-4 phényl)-méthylène-2 succinamique (composé No 13) fondant à 174–175°C. Rendement: 94%,
- N-(méthyl-4 phényl)-méthylène-2 succinamate d'éthanolamine (composé No 14). Liquide visqueux. Rendement: 100%.

Les propriétés régulatrices de la croissance des plantes des composés selon l'invention sont mises en évidence par les essais décrits dans les exemples I à III. Dans ces essais, on entendra par solution, soit une solution aqueuse lorsque la matière active est hydrosoluble, soit, dans le cas contraire, une dispersion ou émulsion aqueuse obtenue en diluant par de l'eau, respectivement, soit une poudre mouillable comprenant en poids 20% de matière active, soit un concentré émulsionnable contenant environ 200 g/l de matière active. Ces essais sont effectués soit en serre, soit en plein champ, en traitant les feuilles de plantes telles que haricot et soja par une solution dont la teneur en matière active à tester peut varier de 0,01 g/l à 20 g/l. On observe ensuite l'évolution biométrique et morphologique dans le temps, des plantes traitées et on la compare à celle de plantes témoins traitées dans les mêmes conditions par une solution ne contenant pas de matière active.

Exemple I

Essai en serre sur haricot (Phaseolus vulgaris)
de variété Contender, traitement de postlevée

On prépare une solution de la matière à tester, à la concentration voulue, en diluant par de l'eau un concentré émulsionnable ayant la composition suivante:

| | |
|---|---|
| — matière à tester | 200 g |
| — agent mouillant et émulsifiant (phosphate acide de nonylphénol oxyéthyléné à 9 oxydes d'éthylène | 100 g |
| — solvant (cyclohexanone) q.s.p | 1 litre |

Dans des pots de 9 × 9 × 9 cm, remplis de terre agricole légère, on sème des graines de haricot que l'on recouvre ensuite par une couche de terre d'un demi centimètre d'épaisseur et les pots sont conservés en serre à température ambiante, sous 70% d'humidité relative et arrosés en subirrigation.

Lorsque les plants de haricot ont atteint le stade deux feuilles primordiales développées et bourgeon terminal prêt à s'ouvrir, on traite leur feuillage par pulvérisation au moyen de la solution préparée précédemment, à raison d'environ 500 l/ha de solution, pour des doses de matière active allant de 1 à 8 kg/ha.

Les pots traités sont ensuite placés dans des bacs destinés à recevoir l'eau d'arrosage en sub-irrigation, puis maintenus pendant 35 jours, à température ambiante sous 70% d'humidité relative. Pendant cette période, on observe les résultats dans le cas des plants traités par la matière active selon l'invention et on compare à ceux obtenus dans le cas de plants-témoins traités par une solution ne contenant pas de matière active.

Dans ces conditions, on observe que, par rapport aux témoins non traités, les plants traités au moyen des composés selon l'invention présentent les effets ci-après:

Composé No 1

A 1 et 2 kg/ha:
réduction de taille de 45% (c'est-à-dire que la hauteur moyenne des plants traités est égale à 55% de celle du plant-témoin non traité).
Aux doses de 4 et 8 kg/ha:
réduction de taille de 60% et développement très important de pousses latérales à partir de bourgeons axillaires.

Composé No 2

A 8 kg/ha:
réduction de taille de 45% et
à 4 kg/ha:
réduction de taille de 30%.
A 8, 4, 2 et 1 kg/ha:
développement très important de pousses latérales à partir des bourgenons axillaires, entraînant une augmentation très nette du nombre de ramifications et faible déformation des feuilles trifoliées nouvellement formées.

Composé No 3

A 8 et 4 kg/ha:
réduction de taille d'environ 25% et faible déformation des feuilles trifoliées.

Composé No 4

A 4 et 8 kg/ha:
réduction de taille d'environ 25% et développement de pousses latérales à partir des bourgeons axillaires légèrement supérieur à celui du plant-témoin.

Composé No 5

A 8 et 4 kg/ha:
importante réduction de taille (c'est-à-dire comprise entre 30 et 60%) et développement important de pousses latérales à partir des bourgeons axillaires.
A 2 kg/ha:
faible développement de pousses latérales.

Composé No 6

A 8 et 4 kg/ha:
réduction de taille d'environ 60% et très important développement de pousses latérales à partir des bourgeons axillaires.
A 2 kg/ha:
réduction de taille de 40% et à 1 kg/ha de 30%.

Composé No 7

A 8 kg/ha:
importante réduction de taille et développement important de pousses latérales à partir des bourgeons axillaires avec légère déformation des feuilles trifoliées.
A 4 kg/ha:
faible réduction de taille (c'est-à-dire inférieure à 30%) et faible développement de pousses latérales à partir des bourgeons axillaires.

Composé No 8

A 8 kg/ha:
faible réduction de taille et développement important de pousses latérales à partir des bourgeons axillaires avec légère déformation des feuilles trifoliées.

Composé No 10

A 8, 4 et 2 kg/ha:
importante déformation des feuilles trifoliées (feuilles ayant l'apparence de cuillères).

Composé No 12

A 8 kg/ha:
réduction de taille de 35% et faible développement de pousses latérales à partir des bourgeons axillaires.

Exemple II

Essai en serre sur haricot de variété Contender, traitement de prélevée

On opère comme à l'exemple précédent si ce n'est qu'on traite sur le sol immédiatement après le semis des graines de haricot.

Dans ces conditions, le plant correspondant au traitement par le composé No 2, à la dose de 8 kg/ha présente par rapport au témoin un développement très important des pousses latérales.

Exemple III

Essai de plein champ sur soja (Glycine max) de variété Amsoy-71

Dans une parcelle de 3 x 10 m, on sème le 27 juillet des graines de soja, selon trois rangées parallèles de 10 m de long chacune, espacées entre elles de 0,50 m.

Le 20 août, lorsque les plants de soja sont au stade deux feuilles trifoliées étalées, on traite les plants

de deux de ces rangées par pulvérisation au moyen d'une solution préparée en diluant par de l'eau, à la concentration voulue, le même concentré émulsionnable qu'à l'exemple I.

Cette solution est appliquée à ruisellement sur des plants de soja, de façon à ce que la dose de matière active appliquée sur deux rangées soit respectivement de 1 kg/ha (première rangée), et de 2 kg/ha (deuxième rangée), la troisième rangée n'étant soumise à aucun traitement de façon à être utilisée comme témoin.

Le 18 décembre, on évalue le nombre de ramifications des plants traités, on récolte les gousses formées et on compare aux résultats obtenus dans le cas des plants-témoins de la troisième rangée.

Dans ces conditions, on observe que les résultats obtenus en utilisant comme matière active le composé No 2 sont les suivants, (les valerus indiquées en ce qui concerne le nombre de ramifications et le nombre de gousses se rapportant à 100 plants de soja).

| Dose de matière active | témoin | composé No 2<br>2 kg/ha | <br>1 kg/ha |
|---|---|---|---|
| Nombre de ramifications (100 plants) | 84 | 114 | 186 |
| Nombre de gousses (100 plants) | 1664 | 2694 | 2815 |
| Poids de grains récoltés (100 plants) | 678 g | 1160 g | 1390 g |
| Poids de 1000 grains | 180 g | 226 g | 220 g |

Dans un autre essai distinct de celui décrit ci-dessus, on opère selon la même méthode que dans cet essai si ce n'est que le traitement est effectué le 30 août, sur les plants de soja au stade six feuilles trifoliées (les graines de soja ayant été semées à la même date que dans l'essai précédent). Les résultats observés le 18 décembre sont rapportés dans le tableau ci-après.

| Dose de matière active utilisée | témoin | composé No 2<br>2 kg/ha | <br>1 kg/ha |
|---|---|---|---|
| Nombre de ramifications (100 plants) | 104 | 117 | 125 |
| Nombre de gousses (100 plants) | 2176 | 2651 | 2871 |
| Poids de grains récoltés (100 plants) | 1005 g | 1303 g | 1279 g |
| Poids de 1000 grains | 162 g | 176 g | 172 g |

On observera d'après ces essais que les traitements effectués au moyen du composé No 2 entraînenent par rapport au témoin non traité une augmentation importante de la récolte de soja.

Ces résultats montrent bien les propriétés remarquables des composés selon l'invention qui peuvent donc être utilisés sur toutes sortes de plantes, monocotylédones (notamment graminées) et dicotylédones, comme celles de grandes cultures (notamment soja), cultures industrielles, fruits, légumes, plantes d'ornement, plantes médicinales et à parfum etc, en vue d'augmenter les rendements, mais aussi pour favoriser la ramification, modifier le port, réduire la taille pour obtenir des plantes plus ramassées etc . . . Comme composés préférés, on peut mentionner tout spécialement les composés No 2, 5, 6, 7, 10 et 1.

Pour leur emploi dans la pratique, les composés selon l'invention sont rarement utilisés seuls. Le plus souvent, ils font partie de compositions qui comprennent, en général, en plus de la matière active selon l'invention, un support et/ou agent tensioactif compatible avec la matière active et utilisable en agriculture ou horticulture.

De façon générale, ces compositions contiennent de 0,001 à 95% en poids de matière active. Leur teneur en agent tensioactif est généralement comprise entre 0 et 20% en poids.

Le terme »support« aus sens de la présente description désigne une matière, organique ou minérale, naturelle ou synthétique, avec laquelle la matière active est associée pour faciliter son application sur la plante, sur des graines ou sur le sol, ou son transport, ou sa manipulation. Le support peut être solide (par exemple argiles, silicates naturels ou synthétiques, carbonates de calcium ou de magnésium, sulfate de calcium) ou liquide (par exemple eau, alcools, cétones, éthers-oxydes, esters, hydrocarbures aromatiques, hydrocarbures halogénés, fractions de pétrole).

Lorsque le support est l'eau ou un solvant organique usuel, la composition comprend de préférence,

en plus de la matière active, un autre composé tel que, par exemple un agent tensioactif, un pesticide (par exemple un insecticide ou fongicide) ou une autre substance régulatrice de la croissance des plantes.

L'agent tensioactif (ou agent de surface), utilisable dans les compositions selon l'invention, peut être un agent mouillant, dispersant ou émulsifiant, pouvant être ionique ou non ionique. On peut citer, par exemple, des non ioniques, comme les condensats d'oxyde d'éthylène avec les alcools, acides gras, amides ou amines grasses ou les alkylphénols, les esters d'acides gras, et du sorbitol, dérivés du saccharose, des anioniques, comme les sels d'acides lignosulfoniques, d'acides alkylarylsulfoniques, d'alcoyl sulfosuccinates et sulfosuccinamates, des dérivés d'aminoacides, des cationiques comme des acétates d'alcoylamines ou d'imidazoline, des amphotères comme les alcoylbétaïnes ou suslfobétaines.

En plus de la matière active, du support et de l'agent tensioactif, les compositions selon l'invention peuvent contenir d'autres additifs tels que des agents dispersants non tensioactifs, des peptisants, colloïdes protecteurs, des épaississants, des adhésifs augmentant la résistance à la pluie, des stabilisants, des agents conservateurs, des inhibiteurs de corrosions, des colorants, des séquestrants, des anti-mousses, anti-mottants, anti-gels etc.

Les compositions selon l'invention peuvent être préparées sous la forme de poudres mouillables, de poudres solubles, de poudres pour poudrage, de granulés, de solutions, de concentrés émulsionnables, d'émulsions, suspensions concentrées ou de suspensions et de dispersions.

Les poudres mouillables contiennent habituellement de 10 à 95% en poids de matière active et elles contiennent habituellement, en plus de la matière active et d'un support inerte, de 0 à 5% en poids d'un agent mouillant, de 3 à 10% en poids d'un agent dispersant, et, lorsque nécessaire, de 0 à 10% en poids de un ou plusieurs stabilisants et/ou d'autres additifs tels que des agents de pénétration, des adhésifs, des agents antimottants ou des colorants.

Elles sont habituellement préparées par pré-mélange de la matière active avec un support solide et des agents mouillants et dispersants. Ce pré-mélange est ensuite broyé soit dans un broyeur, par exemple à broches équipé d'un sélecteur à poudre, soit dans un broyeur »Air Jet«, ce dernier appareil étant préfére pour les matières actives à bas point de fusion.

A titre d'exemples, voici la composition de trois poudres mouillables selon l'invention; les pourcentages étant exprimés en poids:

| | | | |
|---|---|---|---|
| — matière active (composé No 2) | 20% | 50% | 70,0% |
| — alkylnapthalène-sulfonate de sodium (mouillant) | 1% | 1% | 1,5% |
| — lignosulfonate de calcium (défloculant) | 5% | 5% | 6,0% |
| — argile kaolinique (charge inerte) | 74% | 44% | 22,5% |

Les poudres solubles dans l'eau sont habituellement obtenues par mélange de 20 à 95% en poids de matière active, de 0 à 10% d'une charge antimottante et de 0 à 5% d'un agent mouillant, le reste étant constitué par une charge hydrosoluble principalement un sel. Ces poudres peuvent aussi comprendre des agents dispersants et des colloides protecteuers.

Voici un exemple de composition d'une poudre hydrosoluble:

| | |
|---|---|
| — matière active (composé No 7) | 80,0% |
| — agent mouillant anionique (alcoyl-napthalène sulfonate alcalin) | 0,5% |
| — silice antimottante | 4,0% |
| — sulfate d'ammonium | 15,5% |

Les poudres pour poudrages peuvent être préparées comme les poudres mouillables mais sans les tensioactifs, dispersants, colloïdes protecteurs inutiles dans ces formulations. Dans ce cas, le support solide est choisi spécialement pour faciliter le broyage, absorber si la matière active est liquide et garantir la fluence.

Les granulés et micro-granulés, généralement à faible titre de matière active, peuvent être pré-fabriqués puis imprégnés ou fabriqués dans la masse par exemple par atomisation ou par extrusion. Dans ce dernier cas, ils nécessitent l'adjonction de liants et de produits hydroscopiques pour assurer leur délitage rapide au sol. Ils contiennent en général de 0,5 à 25% en poids de matière active, de 0 à 10% en poids d'additifs comme des stabilisatns, des agents de modification à libération lente, des liants et des solvants.

Les concentrés émulsionnables contiennent la matière active dissoute dans un solvant (généralement un hydrocarbure aromatique) et en plus, quand c'est nécessaire, un solvant auxilliaire ou cosolvant pouvant être une cétone, un ester, un éther-oxyde, etc... En général, ils contiennent de 5

à 60% en poids/volume de matières actives, de 2 à 20% en poids/volume d'agent émulsifiant et peuvent être préparés, par exemple, par dissolution de matière active, dans le solvant ou dans le mélange de solvants et émulsifiant, dans une cuve équipée d'une bonne agitation et d'une circulation de fluide pour le chauffage ou le refroidissement.

A titre d'exemple, voici la composition de trois concentrés émulsionnables selon l'invention:

| | | | |
|---|---|---|---|
| — matière active (composé No 2) | 50 g | 100 g | 200 g |
| — phosphate acide de nonylphénol oxyéthyléné à 9 molécules d'oxyde d'éthylène | 30 g | 50 g | 100 g |
| — cyclohexanone | 200 g | 233 g | 467 g |
| — solvant (hydrocarbures aromatiques) q. s. p. | 1 litre | 537 g | 202 g |

Les suspensions concentrées appelées »flowables«, également applicables en pulvérisation après dilution dans l'eau, sont préparées de manière qu'on obtienne un produit fluide, stable, ne se déposant pas. Elles contiennent de 10 à 50% en poids de matière active, de 0,5 à 15% en poids d'agents tensioactifs, de 0,5 à 10% en poids de colloïdes protecteurs ou agents antisédiments, de 0 à 10% d'additifs divers, comme des anti-mousses, épaississants, stabilisants, adhésifs, anti-gels et comme support de l'eau ou un liquide organique dans leqeul la matière active est insoluble ou un mélange des deux. Certaines matières solides organiques ou des sels minéraux peuvent être dissous dans le support pour retarder la sédimentation ou comme anti-gel pour l'eau.

Ces suspensions concentrées peuvent être préparées, par exemple, par dispersion de la matière active pré-broyées dans un support liquide pouvant être de l'eau, une huile végétale ou minérale ou le mélange émulsionné des deux contenant les ingrédients auxilliaires comme l'agent de viscosité, le conservateur et l'antigel, puis broyage de la dispersion dans un broyeur à billes.

Des dispersions et émulsions aqueuses, par exemple des compositions obtenues en diluant à l'aide d'eau une poudre mouillable ou un concentré émulsionnable selon l'invention, sont comprises dans le cadre général de la présente invention. Ces émulsions peuvent être du type eau-dans-l'huile ou du type huile-dans-l'eau, et elles peuvent avoir une consistance épaisse comme celle d'une »mayonnaise«.

Toutes ces compositions peuvent être apportées aux plantes par diverses méthodes comme la pulvérisation sur la partie aérienne des plantes, le trempage des graines, des plantes, des mottes, des racines ou des fruits, l'arrosage du sol, l'injection à la plante etc.

La présente invention concerne enfin un procédé pour modifier la croissance des plantes (c'est-à-dire pour modifier leur physiologie de diverses manières) selon lequel on applique sur les plantes ou dans leur environnement une quantité efficace d'au moins un composé selon la formule (I), ou d'un sel acceptable en agriculture d'un de ces composés. La dose de matière active à tuiliser peut varier selon différents facteurs tels que le type de culture à traiter, son stade de développement, les conditions climatiques, la nature du terrein ... En pratique, les traitements selon l'invention sont effectués en appliquant sur lesdites cultures des doses de matière active pouvant aller de 0,05 kg/ha à 10 kg/ha.

**Revendications pour lés Etats Contractants: BE, CH, DE, FR, GB, IT, LI, LU, NL**

1. Composition régulatrice de la croissance des plantes, caractérisé en ce qu'elle contient comme matière active:

au moins un composé de formule:

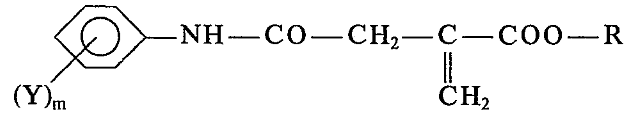

dans laquelle:

R représente un atome d'hydrogène ou un radical alcoyle contenant de 1 à 16 atomes de carbone, ou alcényle contenant de 3 à 15 atomes de carbone ou alcynyle contenant de 3 à 5 atomes de carbone,

Y représente un atome d'halogène ou un radical alcoyle contenant de 1 à 4 atomes de carbone,

m est un nombre entier de 0 à 5, les Y pouvant être identiques ou différents,

ou un sel acceptable en agriculture du composé selon la formule ci-dessus pour lequel R représente l'atome d'hydrogène.

2. Composition selon la revendication 1, caractérisée en ce qu'elle contient comme matière active:

au moins un composé de formule:

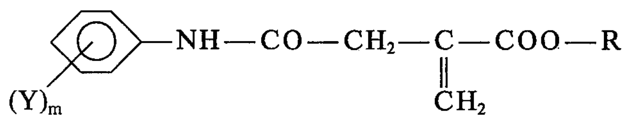

dans laquelle Y représente un atome d'halogène, m est égal à 0, 1, 2 ou 3, les Y pouvant être identiques ou différents et R a même signification que dans la revendication 1,

ou un sel acceptable en agriculture du composé selon la formule ci-dessus pour lequel R représente l'atome d'hydrogène.

3. Composition selon la revendication 2, caractérisée en ce qu'elle contient comme matière active au moins un composé répondant à l'une des formules III et III bis:

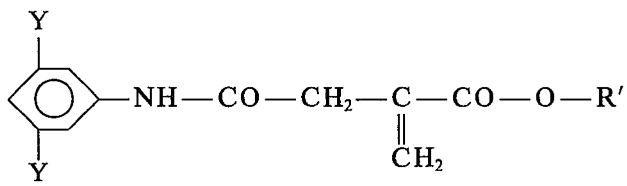

(III)

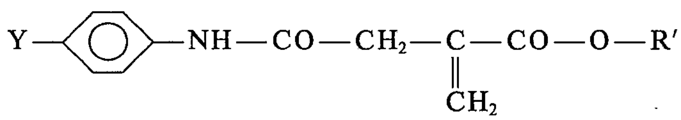

(III bis)

dans lesquelles Y représente un atome d'halogène et R' représente l'atome d'hydrogène ou un radical alcoyle contenant de 1 à 8 atomes de carbone, ou alcényle contenant de 3 à 5 atomes de carbone ou un sel acceptable en agriculture de ce composé pour lequel R' représente l'atome d'hydrogène.

4. Composition selon la revendication 3, caractérisée en ce qu'elle contient comme matière active au moins un composé répondant à l'une des formules III et III bis,

dans lesquelles R' a même signification que dans la revendication 3 et Y représente l'atome de chlore,

ou un sel acceptable en agriculture de ce composé lorsque R' représente l'atome d'hydrogène.

5. Composition selon la revendication 4, caractérisée en ce qu'elle contient comme matière active:

au moins un composé de formule:

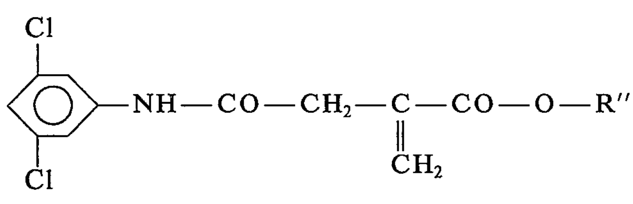

dans laquelle R'' représente l'atome d'hydrogène, ou un radical alcényle comportant de 3 à 5 atomes de carbone,

ou un sel acceptable en agriculture de ce composé lorsque R'' représente l'atome d'hydrogène.

6. Composition selon l'une des revendications 1 à 5, caractérisée en ce que le sel acceptable en agriculture est un sel d'amine.

7. Composition selon l'une des revendications 1 à 6, caractérisée en ce que, en plus de la matière active, elle comprend un support et/ou un agent tensioactif compatible avec la matière active et utilisable en agriculture ou horticulture.

8. Composition selon la revendication 7, caractérisée en ce qu'elle contient de 0,001% à 95% de matière active.

9. Procédé pour modifier la croissance des plantes, caractérisé en ce que l'on applique sur ces plantes ou dans leur environnement une quantité efficace d'au moins un composé de formule:

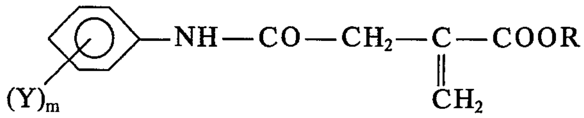

dans laquelle Y, m et R ont même signification que dans la revendicaton 1 ou d'un sel acceptable en agriculture du composé selon la formule ci-dessus pour lequel R représente l'atome d'hydrogène.

10. Procédé selon la revendication 9, caractérisé en ce que le traitement est appliqué sur des cultures de soja.

11. Dérivé de l'acide méthylène-2 succinamique, caractérisé en ce qu'il répond à la formule:

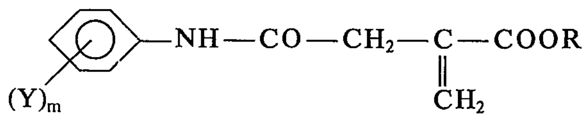

dans laquelle:

- R représente un radical alcoyle contenant de 1 à 16 atomes de carbone, alcényle contenant de 3 à 15 atomes de carbone, alcynyle contenant de 3 à 5 atomes de carbone,
- Y représente un atome d'halogène ou un radical alcoyle contenant de 1 à 4 atomes de carbone,
- m est égal à 0, 1, 2, 3, 4 ou 5, les Y pouvant être identiques ou différents,

et sel acceptable en agriculture du composé selon la formule ci-dessus pour lequel R est remplacé par un atome d'hydrogène.

12. Composé selon la revendication 11, caractérisé en ce que Y représente un atome d'halogène, m est égal à 0, 1, 2 ou 3 et R a même signification que dans la revendication 11, et sel d'amine du composé pour lequel R est remplacé par l'atome d'hydrogène.

13. Composé selon la revendication 12, caractérisé en ce qu'il répond à la formule:

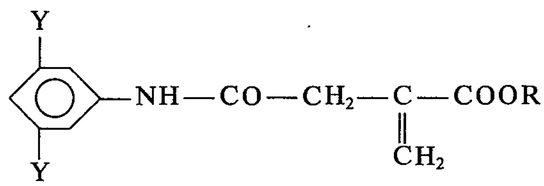

dans laquelle Y représente un atome d'halogene et R représente un radical alcényle comportant de 3 à 5 atomes de carbone.

14. Procédé de préparation d'un composé selon la revendication 11, caracterisé en ce qu'il consiste à faire réagir un acide de formule:

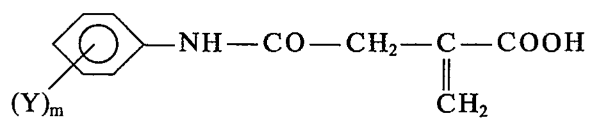

dans laquelle Y et m même signification que dans la revendication 11, sur un alcool de formule ROH dans laquelle R à même signification que dans la revendication 11.

15. Procédé de préparation d'un composé selon la revendication 11, caractérisé en ce qu'il consiste à faire réagir un sel de l'acide de formule:

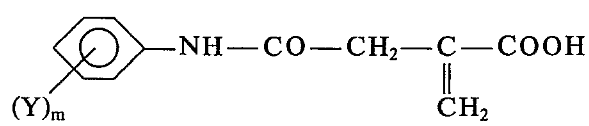

dans laquelle Y et m ont même signification que dans la revendication 11, sur un composé de formule XR dans laquelle R a même signification que dans la revendication 11 et X représente un atome d'halogène.

**Revendications pour l'Etat Contractant: AT**

1. Composition régulatrice de la croissance des plantes, caractérisée en ce qu'elle contient comme matière active:

au moins un composé de formule:

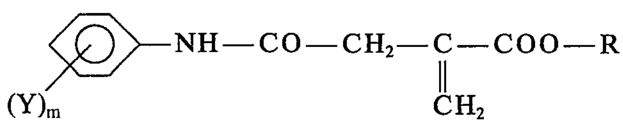

dans laquelle:

R représente un atome d'hydrogène ou un radical alcoyle contenant de 1 à 16 atomes de carbone, ou alcényle contenant de 3 à 15 atomes de carbone ou alcynyle contenant de 3 à 5 atomes de carbone,
Y représente un atome d'halogène ou un radical alcoyle contenant de 1 à 4 atomes de carbone,
m est un nombre entier de 0 à 5, les Y pouvant être identiques ou différents,

ou un sel acceptable en agriculture du composé selon la formule ci-dessus pour lequel R représente l'atome d'hydrogène.

2. Composition selon la revendication 1, caractérisée en ce qu'elle contient comme matière active:

au moins un composé de formule:

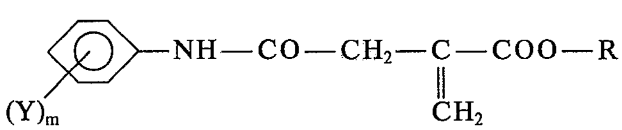

dans laquelle Y représente un atome d'halogène, m est égal à 0, 1, 2 ou 3, les Y pouvant être identiques ou différents et R a même signification que dans la revendication 1,

ou un sel acceptable en agriculture du composé selon la formule ci-dessus pour lequel R représente l'atome d'hydrogène.

3. Composition selon la revendication 2, caractérisée en ce qu'elle contient comme matière active au moins un composé répondant à l'une des formules III et III bis:

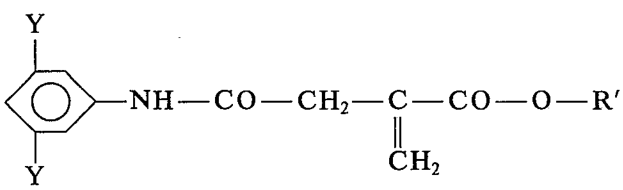

(III)

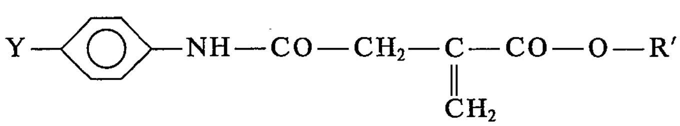

(III bis)

dans lesquelles Y représente un atome d'halogène et R' représente l'atome d'hydrogène ou un radical alcoyle contenant de 1 à 8 atomes de carbone, ou alcényle contenant de 3 à 5 atomes de carbone ou un sel acceptable en agriculture de ce composé pour lequel R' représente l'atome d'hydrogène.

4. Composition selon la revendication 3, caractérisée en ce qu'elle contient comme matière active au moins un composé répondant à l'une des formules III et III bis,

dans lesquelles R' a même signification que dans la revendication 3 et Y représente l'atome de chlore,

ou un sel acceptable en agriculture de ce composé lorsque R' représente l'atome d'hydrogène.

5. Composition selon la revendication 4, caractérisée en ce qu'elle contient comme matière active au moins un composé de formule:

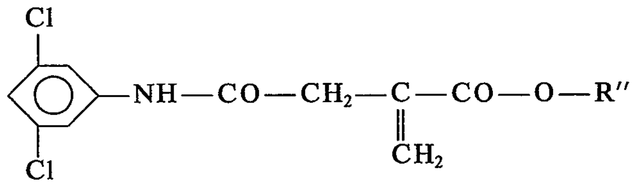

dans laquelle R″ représente l'atome d'hydrogène, cu un radical alcényle comportant de 3 à 5 atomes de carbone,

ou un sel acceptable en agriculture de ce composé lorsque R″ représente l'atome d'hydrogène.

6. Composition selon l'une des revendications 1 à 5, caractérisée en ce que le sel acceptable en agriculture est un sel d'amine.

7. Composition selon l'une des revendications 1 à 6, caractérisée en ce que, en plus de la matière active, elle comprend un support et/ou un agent tensioactif compatible avec la matière active et utilisable en agriculture ou horticulture.

8. Composition selon la revendication 7, caractérisée en ce qu'elle contient de 0,001 à 95% de matière active.

9. Procédé pour modifier la croissance des plantes, caractérisé en ce que l'on applique sur ces plantes ou dans leur environnement une quantité efficace d'au moins un composé de formule:

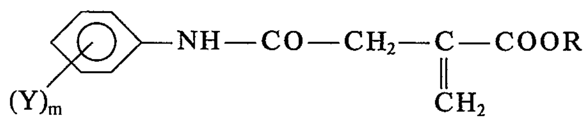

dans laquelle Y, m et R ont même signification que dans la revendication 1 ou d'un sel acceptable en agriculture du composé selon la formule ci-dessus pour lequel R représente l'atome d'hydrogène.

10. Procédé selon la revendication 9, caractérisé en ce que le traitement est appliqué sur des cultures de soja.

11. Procédé de préparation d'un composé de formule:

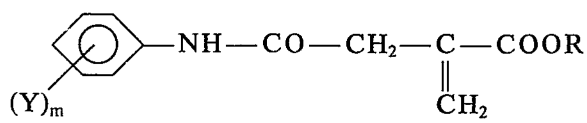

dans laquelle Y et m ont la même signification que dans la revendication 1 et R représente un radical alcoyle contenant de 1 à 16 atomes de carbone, alcényle contenant de 3 à 15 atomes de carbone, ou alcynyle contenant de 3 à 5 atomes de carbone, caractérisé en ce qu'il consiste à faire réagir l'acide de formule:

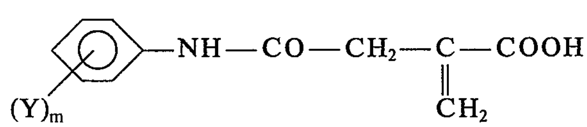

dans laquelle Y et m ont même signification que précédemment, sur un alcool de formule ROH dans laquelle R a même signification que précédemment.

12. Procéde de préparation d'un composé de formule:

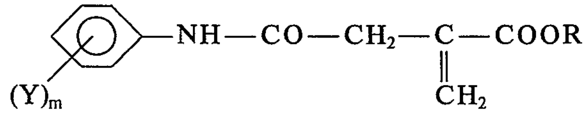

dans laquelle Y, m et R ont la même signification que dans la revendication 11, caractérisé en ce qu'il consiste à faire réagir un sel de l'acide de formule:

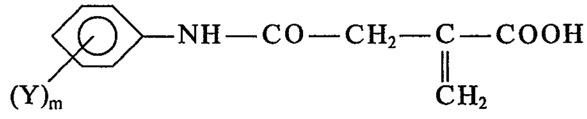

dans laquelle Y et m ont même signification que précédemment, sur un composé de formule XR dans laquelle R a même signification que précédemment et X représente un atome d'halogène.

**Patentansprüche für die Vertragsstaaten: BE, CH, DE, FR, GB, IT, LI, LU, NL**

1. Zusammensetzung mit Wachstumsregulierung für Pflanzen, dadurch gekennzeichnet, daß sie als aktives Material enthält:

mindestens eine Verbindung der Formel

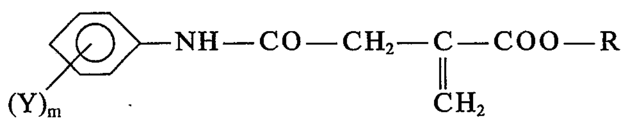

worin

R ein Wasserstoffatom oder einen Alkylrest mit 1 bis 16 Kohlenstoffatomen oder Alkenylrest mit 3 bis 15 Kohlenstoffatoamen oder Alkinylrest mit 3 bis 5 Kohlenstoffatomen,
Y ein Halogenatom oder einen Alkylrest mit 1 bis 4 Kohlenstoffatomen bedeutet und
m eine ganze Zahl von 0 bis 5 ist, wobei die Reste Y identisch oder voneinander verschieden sein können;

oder ein in der Landwirtschaft annehmbares Salz der Verbindung gemäß obiger Formel, worin R ein Wasserstoffatom ist.

2. Zusammensetzung gemäß Anspruch 1, dadurch gekennzeichnet, daß sie als aktives Material enthält:

mindestens eine Verbindung der Formel

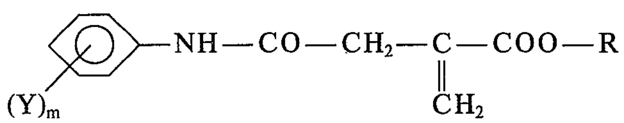

worin Y ein Halogenatom bedeutet, m gleich 0, 1, 2 oder 3 ist, wobei die Reste Y identisch oder voneinander verschieden sein können, und R dieselbe Bedeutung wie in Anspruch 1 hat;

oder ein in der Landwirtschaft annehmbares Salz der Verbindung gemäß obiger Formel, worin R ein Wasserstoffatom ist.

3. Zusammensetzung gemäß Anspruch 2, dadurch gekennzeichnet, daß sie als aktives Material mindestens eine Verbindung entsprechend einer der Formeln III und III bis

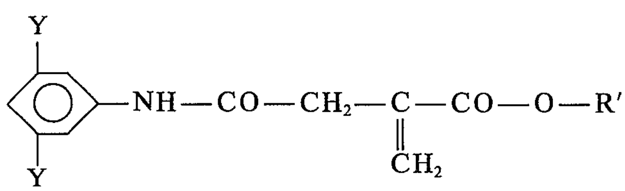

(III)

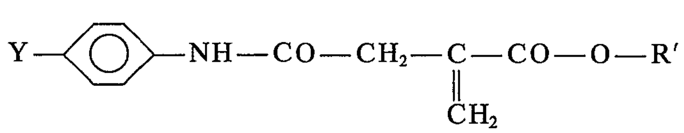

(III bis)

enthält, worin Y ein Halogenatom bedeutet und R′ das Wasserstoffatom oder einen Alkylrest mit 1 bis 8 Kohlenstoffatomen oder Alkenylrest mit 3 bis 5 Kohlenstoffatomen bedeutet oder ein in der Landwirtschaft annehmbares Salz dieser Verbindung, wofür R′ das Wasserstoffatom bedeutet.

4. Zusammensetzung gemäß Anspruch 3, dadurch gekennzeichnet, daß sie als aktives Material mindestens eine Verbindung entsprechend einer der Formeln III und III bis enthält, worin R′ dieselbe Bedeutung wie in Anspruch 3 hat und Y das Chloratom bedeutet, oder ein in der Landwirtschaft annehmbares Salz dieser Verbindung, wenn R′ das Wasserstoffatom bedeutet.

5. Zusammensetzung gemäß Anspruch 4, dadurch gekennzeichnet, daß sie als aktives Material mindestens eine Verbindung der Formel

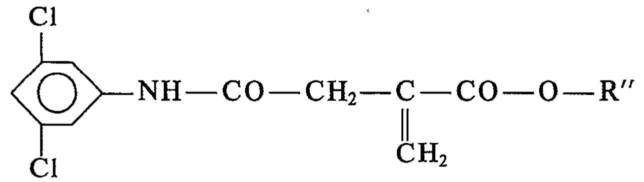

enthält, worin R″ das Wasserstoffatom oder einen Alkenylrest mit 3 bis 5 Kohlenstoffatomen bedeutet, oder ein in der Landwirtschaft annehmbares Salz dieser Verbindung, wenn R″ das Wasserstoffatom bedeutet.

6. Zusammensetzung gemäß einem der Ansprüche 1 bis 5, dadurch gekennzeichnet, daß das in der Landwirtschaft annehmbare Salz ein Aminsalz ist.

7. Zusammensetzung gemäß einem der Ansprüche 1 bis 6, dadurch gekennzeichnet, daß sie außer dem aktiven Material einen Träger und/oder ein oberflächenaktives Mittel umfaßt, das mit dem aktiven Material verträglich und in der Landwirtschaft oder Gärtnerei verwendbar ist.

8. Zusammensetzung gemäß Anspruch 7, dadurch gekennzeichnet, daß sie 0,001% bis 95% aktives Material enthält.

9. Verfahren zum Modifizieren des Wachstums der Pflanzen, dadurch gekennzeichnet, daß man auf diese Pflanzen oder in ihrer Umgebung eine wirksame Menge wenigstens einer Verbindung der Formel

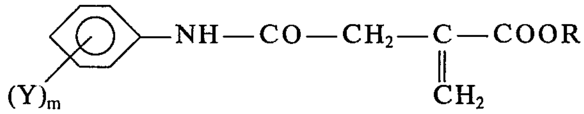

worin Y, m und R dieselbe Bedeutung wie in Anspruch 1 haben, oder ein in der Landwirtschaft annehmbares Salz der Verbindung gemäß obiger Formel, worin R ein Wasserstoffatom bedeutet, aufbringt.

10. Verfahren gemäß Anspruch 9, dadurch gekennzeichnet, daß die Behandlung auf Sojakulturen angewandt wird.

11. Derivat der 2-Methylen-succinamidsäure, dadurch gekennzeichnet, daß es der Formel

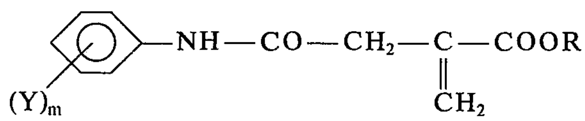

entspricht, worin

R einen Alkylrest mit 1 bis 16 Kohlenstoffatomen, einen Alkenylrest mit 3 bis 15 Kohlenstoffatomen, einen Alkinylrest mit 3 bis 5 Kohlenstoffatomen bedeutet,
Y ein Halogenatom oder einen Alkylrest mit 1 bis 4 Kohlenstoffatomen bedeutet,
m gleich 0, 1, 2, 3, 4 oder 5 ist, wobei die Reste Y identisch oder voneinander verschieden sein können;

und ein in der Landwirtschaft annehmbares Salz der Verbindung der obigen Formel, worin R ein Wasserstoffatom darstellt.

12. Verbindung gemäß Anspruch 11, dadurch gekennzeichnet, daß Y ein Halogenatom bedeutet, m gleich 0, 1, 2 oder 3 ist und R dieselbe Bedeutung wie in Anspruch 11 hat, und das Aminsalz der Verbindung, worin R durch ein Wasserstoffatom ersetzt ist.

13. Verbindung gemäß Anspruch 12, dadurch gekennzeichnet, daß sie der Formel

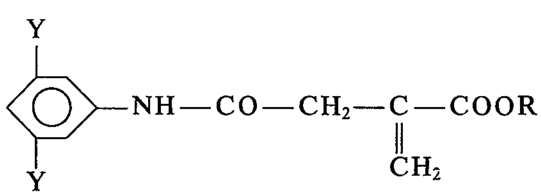

entspricht, worin Y ein Halogenatom darstellt und R einen Alkenylrest mit 3 bis 5 Kohlenstoffatomen bedeutet.

14. Verfahren zur Herstellung einer Verbindung gemäß Anspruch 11, dadurch gekennzeichnet, daß man eine Säure der Formel

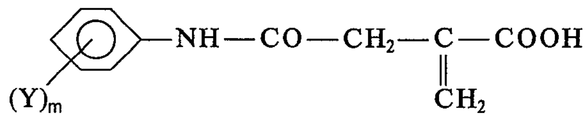

worin Y und m dieselbe Bedeutung wie im Anspruch 11 haben, auf einen Alkohol der Formel ROH einwirken läßt, worin R dieselbe Bedeutung wie im Anspruch 11 hat.

15. Verfahren zur Herstellung einer Verbindung gemäß Anspruch 11, dadurch gekennzeichnet, daß man ein Salz der Säure der Formel

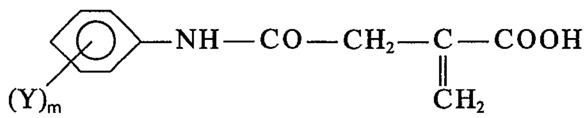

worin Y und m dieselbe Bedeutung wie in Anspruch 11 haben, auf eine Verbindung der Formel XR, worin R dieselbe Bedeutung wie in Anspruch 11 hat und X ein Halogenatom bedeutet, einwirken läßt.

**Patentansprüche für den Vertragsstaat: AT**

1. Zusammensetzung mit Wachstumsregulierung für Pflanzen, dadurch gekennzeichnet, daß sie als aktives Material enthält:

mindestens eine Verbindung der Formel

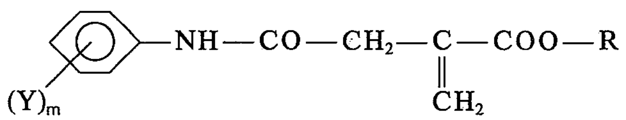

worin

R ein Wasserstoffatom oder einen Alkylrest mit 1 bis 16 Kohlenstoffatomen oder Alkenylrest mit 3 bis 15 Kohlenstoffatomen oder Alkinylrest mit 3 bis 5 Kohlenstoffatomen,
Y ein Halogenatom oder einen Alkylrest mit 1 bis 4 Kohlenstoffatomen bedeutet und
m eine ganze Zahl von 0 bis 5 ist, wobei die Reste Y identisch oder voneinander verschieden sein können;

oder ein in der Landwirtschaft annehmbares Salz der Verbindung gemäß obiger Formel, worin R ein Wasserstoffatom bedeutet.

2. Zusammensetzung gemäß Anspruch 1, dadurch gekennzeichnet, daß sie als aktives Material enthält:

mindestens eine Verbindung der Formel

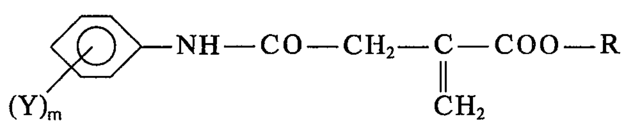

worin Y ein Halogenatom bedeutet, m gleich 0, 1, 2 oder 3 ist, wobei die Reste Y identisch oder voneinander verschieden sein können, und R dieselbe Bedeutung wie in Anspruch 1 hat;

oder ein in der Landwirtschaft annehmbares Salz der Verbindung gemäß obiger Formel, worin R ein Wasserstoffatom bedeutet.

3. Zusammensetzung gemäß Anspruch 2, dadurch gekennzeichnet, daß sie als aktives Material mindestens eine Verbindung entsprechend einer der Formeln III und III bis

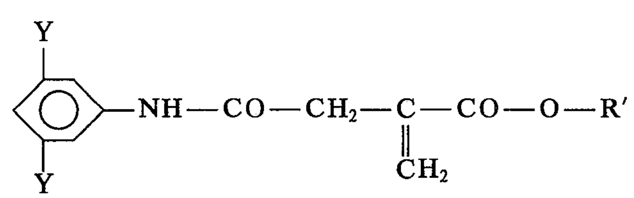

(III)

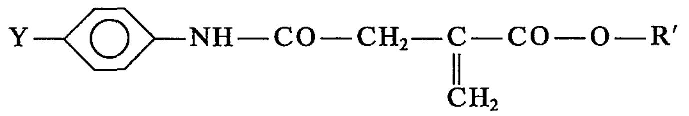

(III bis)

enthält, worin Y ein Halogenatom bedeutet und R′ das Wasserstoffatom oder einen Alkylrest mit 1 bis 8 Kohlenstoffatomen oder Alkenylrest mit 3 bis 5 Kohlenstoffatomen bedeutet, oder ein in der Landwirtschaft annehmbares Salz dieser Verbindung, worin R′ das Wasserstoffatom bedeutet.

4. Zusammensetzung gemäß Anspruch 3, dadurch gekennzeichnet, daß sie als aktives Material mindestens eine Verbindung entsprechend einer der Fomeln III und III bis enthält, worin R′ dieselbe Bedeutung wie in Anspruch 3 hat und Y das Chloratom bedeutet, oder ein in der Landwirtschaft annehmbares Salz dieser Verbindung, wenn R′ das Wasserstoffatm bedeutet.

5. Zusammensetzung gemäß Anspruch 4, dadurch gekennzeichnet, daß sie als aktives Material mindestens eine Verbindung der Formel

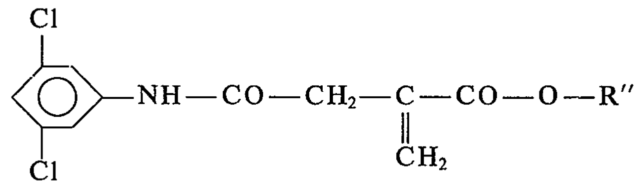

enthält, worin R″ das Wasserstoffatom oder einen Alkenylrest mit 3 bis 5 Kohlenstoffatomen bedeutet, oder ein in der Landwirtschaft annehmbares Salz dieser Verbindung, wenn R″ das Wasserstoffatom bedeutet.

6. Zusammensetzung gemäß einem der Ansprüche 1 bis 5, dadurch gekennzeichnet, daß das in der Landwirtschaft annehmbare Salz ein Aminsalz ist.

7. Zusammensetzung gemäß einem der Ansprüche 1 bis 6, dadurch gekennzeichnet, daß sie außer dem aktiven Material einen Träger und/oder ein oberflächenaktives Mittel umfaßt, das mit dem aktiven Material verträglich und in der Landwirtschaft oder Gärtnerei verwendbar ist.

8. Zusammensetzung gemäß Anspruch 7, dadurch gekennzeichnet, daß sie 0,001 bis 95% aktives Material enthält.

9. Verfahren zum Modifizieren des Wachstums der Pflanzen, dadurch gekennzeichnet, daß man auf diese Pflanzen oder in ihrer Umgebung eine wirksame Menge wenigstens einer Verbindung der Formel

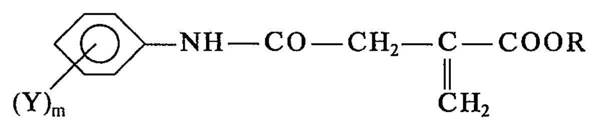

worin Y, m und R dieselbe Bedeutung wie in Anspruch 1 haben, oder ein in der Landwirtschaft annehmbares Salz der Verbindung gemäß obiger Formel, worin R das Wasserstoffatom bedeutet, aufbringt.

10. Verfahren gemäß Anspruch 9, dadurch gekennzeichnet, daß die Behandlung auf Sojakulturen angewandt wird.

11. Verfahren zur Herstellung einer Verbindung der Formel

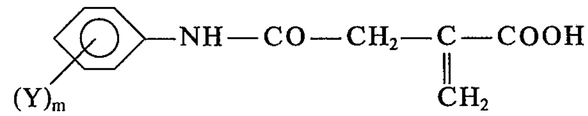

worin Y und m dieselbe Bedeutung wie in Anspruch 1 haben und R einen Alkylrest mit 1 bis 16 Kohlenstoffatomen, einen Alkenylrest mit 3 bis 15 Kohlenstoffatomen oder einen Alkinylrest mit 3 bis 5 Kohlenstoffatomen bedeutet, dadurch gekennzeichnet, daß es darin besteht, eine Säure der Formel

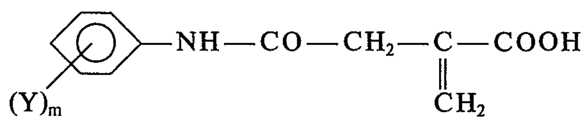

worin Y und m dieselbe Bedeutung wie vorstehend haben, auf einen Alkohol der Formel ROH einwirken läßt, worin R die vorstehend angegebene Bedeutung hat.

12. Verfahren zur Herstellung einer Verbindung der Formel

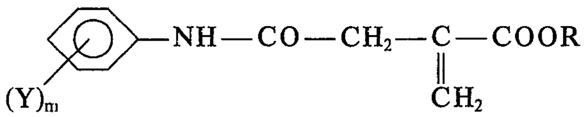

worin Y, m und R dieselbe Bedeutung wie im Anspruch 11 haben, dadurch gekennzeichnet, daß man ein Salz der Säure der Formel

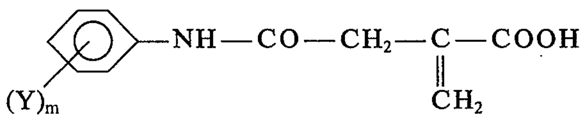

worin Y und m dieselben Bedeutungen wie vorstehend haben, auf eine Verbindung der Formel XR, worin R dieselbe Bedeutung wie vorstehend hat und X ein Halogenatom bedeutet, einwirken läßt.

**Claims for the contracting states: BE, CH, DE, FR, GB, IT, LI, LU, NL**

1. Plant growth regulator composition, characterised in that it contains, as the active material:

at least one compound of the formula:

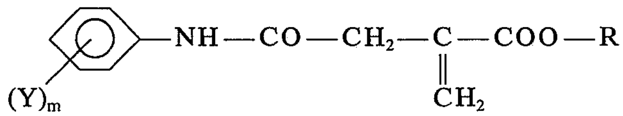

in which:

R represents a hydrogen atom or an alkyl radical containing from 1 to 16 carbon atoms, an alkenyl radical containing from 3 to 15 carbon atoms or an alkynyl radical containing from 3 to 5 carbon atoms,
Y represents a halogen atom or an alkyl radical containing from 1 to 4 carbon atoms,
m is an integer from 0 to 5, and the Y's can be identical or different,

or an agriculturally acceptable salt of the compound according to the above formula, in which R represents a hydrogen atom.

2. Composition according to Claim 1, characterised in that it contains, as the active material:

at least one compound of the formula:

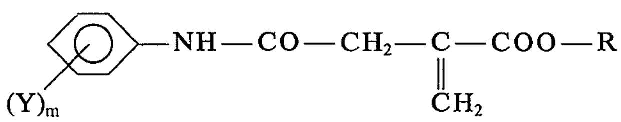

in which Y represents a halogen atom, m is 0, 1, 2 or 3, the Y's can be identical or different and R has the same meaning as in Claim 1,

or an agriculturally acceptable salt of the compound according to the above formula, in which R represents a hydrogen atom.

3. Composition according to Claim 2, characterised in that it contains, as the active material, at least one compound corresponding to one of the formulae III and III bis:

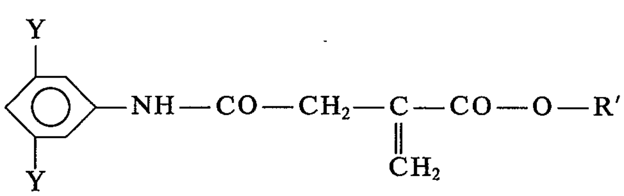
(III)

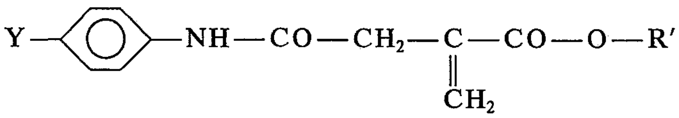
(III bis)

in which Y represents a halogen atom and R′ represents a hydrogen atom or an alkyl radical containing from 1 to 8 carbon atoms or an alkenyl radical containing from 3 to 5 carbon atoms, or an agriculturally acceptable salt of this compound, in which R′ represents a hydrogen atom.

4. Composition according to Claim 3, characterised in that it contains, as the active material, at least one compound corresponding to one of the formulae III and III bis, in which R′ has the same meaning as in Claim 3 and Y represents a chlorine atom, or an agriculturally acceptable salt of this compound, if R′ represents a hydrogen atom.

5. Composition according to Claim 4, characterised in that it contains, as the active material, at least one compound of the formula:

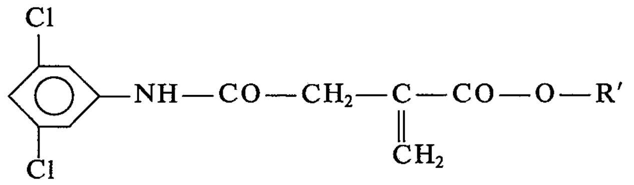

in which R″ represents a hydrogen atom or an alkenyl radical containing from 3 to 5 carbon atoms, or an agriculturally acceptable salt of this compound, if R″ represents a hydrogen atom.

6. Composition according to one of Claims 1 to 5, characterised in that the agriculturally acceptable salt is an amine salt.

7. Composition according to one of Claims 1 to 6, characterised in that in addition to the active material it contains a carrier and/or surfactant compatible with the active material and usable in agriculture or horticulture.

8. Composition according to Claim 7, characterised in that it contains from 0.001% to 95% of active material.

9. Process for modifying plant growth, characterised in that an effective amount of at least one compound of the formula:

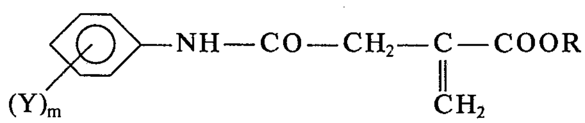

in which Y, m and R have the same meaning as in Claim 1, or of an agriculturally acceptable salt of the compound according to the above formula in which R represents a hydrogen atom, is applied to these plants or to their environment.

10. Process according to Claim 9, characterised in that the treatment is applied to soya cultures.

11. 2-Methylene-succinamic acid derivative, characterised in that it corresponds to the formula:

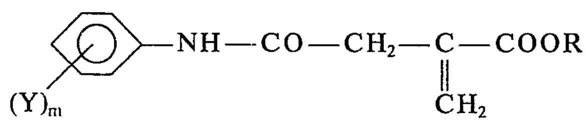

in which:

R represents an alkyl radical containing from 1 to 16 carbon atoms, an alkenyl radical containing from 3 to 15 carbon atoms or an alkynyl radical containing from 3 to 5 carbon atoms,
Y represents a halogen atom or an alkyl radical containing from 1 to 4 carbon atoms,
m is an integer from 0 to 5, and the Y's can be identical or different,

and an agriculturally acceptable salt of the compound according to the above formula, in which R is replaced by a hydrogen atom.

12. Compound according to Claim 11, characterised in that Y represents a halogen atom, m is 0, 1, 2 or 3 and R has the same meaning as in Claim 11, and an amine salt of the compound in which R is replaced by a hydrogen atom.

13. Compound according to Claim 12, characterised in that it corresponds to the formula:

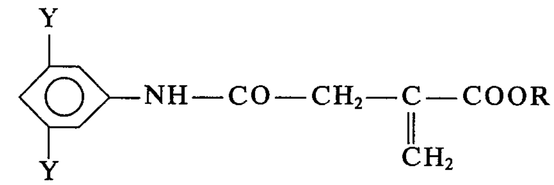

in which Y represents a halogen atom and R represents an alkenyl radical containing from 3 to 5 carbon atoms.

14. Process for the preparation of a compound according to Claim 11, characterised in that it consists in reacting an acid of the formula:

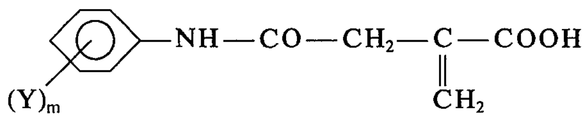

in which Y and m have the same meaning as in Claim 11, with an alcohol of the formula ROH, in which R has the same meanings as in Claim 11.

15. Process for the preparation of a compound according to Claim 11, characterised in that it consists in reacting a salt of the acid of the formula:

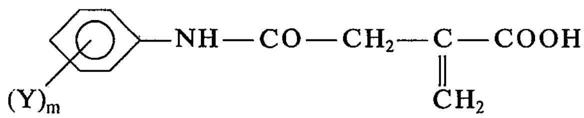

in which Y and m have the same meaning as in Claim 11, with a compound of the formula XR, in which R has the same meaning as in Claim 11 and X represents a halogen atom.

**Claims for the contracting state: AT**

1. Plant growth regulator composition, characterised in that it contains, as the active material:

at least one compound of the formula:

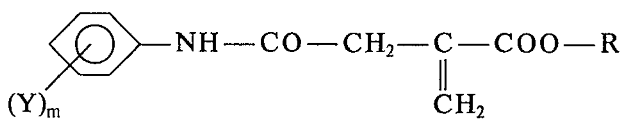

in which:

R represents a hydrogen atom or an alkyl radical containing from 1 to 16 carbon atoms, an alkenyl radical containing from 3 to 15 carbon atoms or an alkynyl radical containing from 3 to 5 carbon atoms,
Y represents a halogen atom or an alkyl radical containing from 1 to 4 carbon atoms,
m is an integer from 0 to 5, and the Y's can be identical or different,

or an agriculturally acceptable salt of the compound according to the above formula, in which R represents a hydrogen atom.

2. Composition according to Claim 1, characterised in that it contains, as the active material:

at least one compound of the formula:

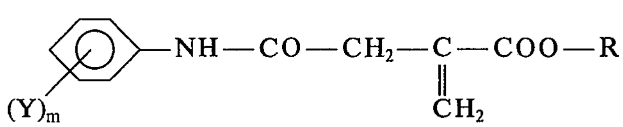

in which Y represents a halogen atom, m is 0, 1, 2 or 3, the Y's can be identical or different and R has the same meaning as in Claim 1,

or an agriculturally acceptable salt of the compound according to the above formula, in which R represents a hydrogen atom.

3. Composition according to Claim 2, characterised in that it contains, as the active material, at least one compound corresponding to one of the formulae III and III bis:

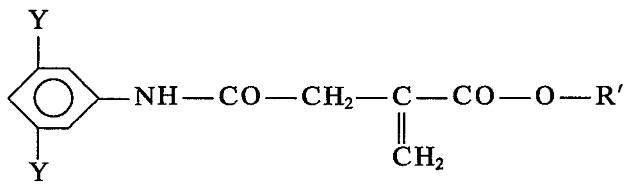

(III)

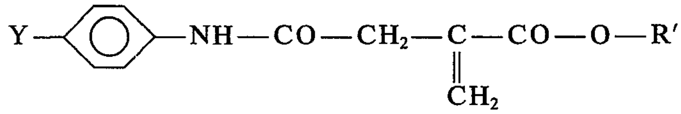

(III bis)

in which Y represents a halogen atom and R′ represents a hydrogen atom or an alkyl radical containing from 1 to 8 carbon atoms or an alkenyl radical containing from 3 to 5 carbon atoms, or an agriculturally acceptable salt of this compound, in which R′ represents a hydrogen atom.

4. Composition according to Claim 3, characterised in that it contains, as the active material, at least one compound corresponding to one of the formulae III and III bis, in which R′ has the same meaning as in Claim 3 and Y represents a chlorine atom, or an agriculturally acceptable salt of this compound, if R′ represents a hydrogen atom.

5. Composition according to Claim 4, characterised in that it contains, as the active material, at least one compound of the formula:

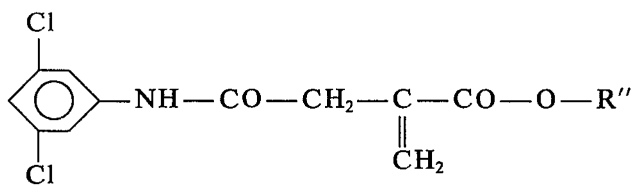

in which R″ represents a hydrogen atom or an alkenyl radical containing from 3 to 5 carbon atoms, or an agriculturally acceptable salt of this compound, if R″ represents a hydrogen atom.

6. Composition according to one of Claims 1 to 5, characterised in that the agriculturally acceptable salt is an amine salt.

7. Composition according to one of Claims 1 to 6, characterised in that in addition to the active material it contains a carrier and/or surfactant compatible with the active material and usable in agriculture or horticulture.

8. Composition according to Claim 7, characterised in that it contains from 0.001% to 95% of active material.

9. Process for modifying plant growth, characterised in that an effective amount of at least one compound of the formula:

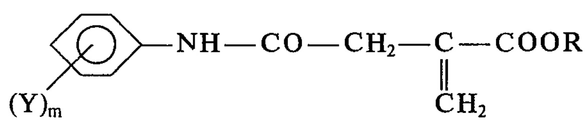

in which Y, m and R have the same meaning as in Claim 1, or of an agriculturally acceptable salt of the compound according to the above formula in which R represents a hydrogen atom, is applied to these plants or to their environment.

10. Process according to Claim 9, characterised in that the treatment is applied to soya cultures.

11. Process for the preparation of a compound of the formula:

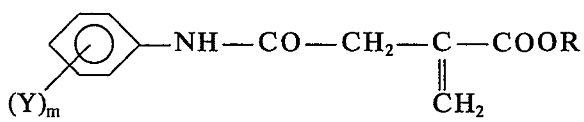

in which Y and m have the same meaning as in Claim 1 and R represents an alkyl radical containing from 1 to 16 carbon atoms, an alkenyl radical containing from 3 to 15 carbon atoms or an alkynyl radical containing from 3 to 5 carbon atoms, characterised in that it consists in reacting the acid of the formula:

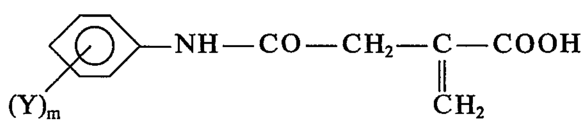

in which Y and m have the same meaning as above, with an alcohol of the formula ROH, in which R has the same meaning as above.

12. Process for the preparation of a compound of the formula:

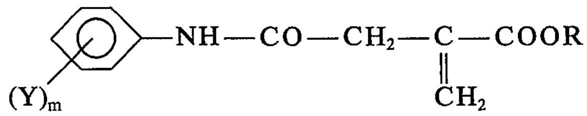

in which Y, m and R have the same meaning as in Claim 11, characterised in that it consists in reacting a salt of the acid of the formula:

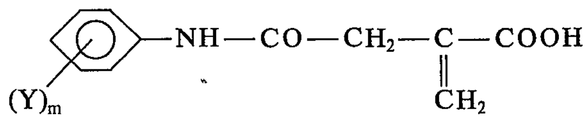

in which Y and m have the same meaning as above, with a compound of the formula XR, in which R has the same meaning as above and X represents a halogen atom.